# FASCICULE DE BREVET EUROPEEN

(11) **EP 2 265 279 B1**
(45) Date de publication et mention de la délivrance du brevet: **22.10.2014**
(21) Numéro de dépôt: 09761861.5
(22) Date de dépôt: 15.04.2009
(51) Int. Cl.: A61K 38/08, A61P 17/06, A61P 19/02, A61P 37/00

(54) **UTILISATION DU NONAPEPTIDE PAT DANS LE TRAITEMENT DE MALADIES AUTOIMMUNES**
VERWENDUNG DES NONAPEPTIDS PAT ZUR BEHANDLUNG VON AUTOIMMUNKRANKHEITEN
USE OF THE NONAPEPTIDE PAT IN THE TREATMENT OF AUTOIMMUNE DISEASES

(30) Priorité: 21.04.2008 FR 0802220
(43) Date de publication de la demande: 29.12.2010
(73) Titulaire: Cll Pharma, 06299 Nice cedex 3 (FR)
(72) Inventeur: TEMSAMANI, Jamal, F-30900 Nîmes (FR); LARUELLE, Claude, F-06299 Nice Cedex 3 (FR)
(74) Mandataire: Peaucelle, Chantal
(86) Numéro de dépôt international: PCT/FR2009/000439
(87) Numéro de publication internationale: WO 2009/150310

(56) Documents cités:
- FR-A- 2 513 125
- FR-A- 2 830 451
- US-A- 4 098 777
- US-A- 4 133 804
- US-A- 4 301 065

## Description

L'invention se rapporte à l'utilisation du nonapeptide PAT pour la fabrication d'un médicament dans le traitement de maladies autoimmunes

Les maladies autoimmunes sont dues à un dysfonctionnement du système immunitaire qui reconnaît de façon inappropriée des constituants du soi conduisant à des réponses immunitaires anormales. L'organisme est « attaqué » par son propre système immunitaire. Aujourd'hui la liste des maladies autoimmunes est exhaustive et comprend entre autres: les maladies inflammatoires chroniques intestinales telles que la maladie de Crohn et la rectocolite hémorragique ; la sclérose en plaques ; l'arthrite psoriasique ; la maladie de Basedow ; la polyarthrite rhumatoïde ; le lupus érythémateux disséminé ; le diabète par insulinorésistance, la sondylarthrite, etc. Parmi les maladies autoimmunes qui représentent un besoin médical urgent, nous allons décrire de manière descriptive mais non limitative : la maladie de Crohn, la polyarthrite rhumatoïde, la rectocolite hémorragique, la sclérose en plaques et le psoriasis. Dans la plupart des maladies autoimmunes telles que la maladie de Crohn, la polyarthrite, etc., certaines cytokines comme le TNF α induisent une attaque du tissu sain qui produit des lésions diverses caractéristiques de chaque pathologie.

Les maladies inflammatoires chroniques de l'intestin (MICI) sont des maladies caractérisées par une inflammation chronique qui touche les intestins. Le mécanisme physiopathologique commun aux MICI est l'inflammation de la muqueuse intestinale. Cette inflammation est créée par une activation du système immunitaire intestinal qui est déclenchée à la fois par des facteurs génétiques et par des facteurs environnementaux. Ce groupe des MICI correspond à deux grandes maladies chroniques, récurrentes qui évoluent par poussées et qui ont tendance à perdurer toute la vie:
- la maladie de Crohn qui peut toucher l'ensemble du tube digestif pratiquement de la bouche jusqu'à l'anus et préférentiellement sur la partie finale de l'intestin grêle et sur le début du côlon. Le diagnostic est basé sur la diarrhée chronique, les douleurs abdominales, les fistules ou abcès péri-anaux, l'amaigrissement et la fièvre. La continuation de l'inflammation dans la maladie de Crohn et la colite ulcérative peut être régulée en partie par une sécrétion accrue de cytokines pro-inflammatoires (Reinecker HC et al., Clin. Exp. Immunol 1993 ;94 :174-181). Selon le même article, le dosage des cytokines pro-inflammatoires TNF-α, Il-1-β et IL-6 à partir de biopsies du colon représente une méthode sensible pour contrôler la sévérité de l'inflammation mucosale chez des patents atteints par une MICI ;
- la rectocolite hémorragique (RCH) qui elle, est localisée uniquement au rectum et au côlon.

Face aux formes sévères de MICI, différents traitements permettent aujourd'hui aux patients de traiter les poussées symptomatiques et dans une moindre mesure de les espacer comme les anti-inflammatoires ; les corticoïdes ; les immunosuppresseurs ; les biothérapies et la, chirurgie. Parmi les traitements anti-inflammatoires, on distingue les dérivés de salicylés, qui induisent le moins de toxicité. La mésalazine est, selon une pratique courante, le traitement que l'on commence à administrer lors d'une première poussée inflammatoire. Ensuite on passe à des anti-inflammatoires plus puissants mais aussi plus toxiques comme la cortisone. Lorsque ces traitements se révèlent inefficaces on a recours parfois à des thérapeutiques plus récentes comme pour la maladie de Crohn, un nouveau traitement qui s'appelle le Remicade®, anticorps monoclonal contre le TNF-α. C'est un médicament très ciblé mais aussi très coûteux qui peut, par exemple, traiter des poussées résistantes au traitement par la cortisone, et maintenir en rémission la maladie de Crohn. Comme traitement d'entretien, pour éviter que le malade ne rechute, on utilise les salicylés et les immuno-suppresseurs qui dépriment l'immunité ; cette dernière étant suractivée lors de ces maladies indiquées ci-dessus. Il faut malheureusement souvent recourir à la chirurgie chez les malades atteintes par ces maladies: plus d'un malade sur deux atteint par la maladie de Crohn sera opéré après huit à dix ans d'évolution, d'une part parce qu'il apparaît souvent des rétrécissements dans l'intestin, parfois également des abcès qui peuvent être localisés autour de la région de l'anus dans 20% des cas environ. Chez les patients atteints par la rectocolite hémorragique qui résistent au traitement, on a alors recours à la chirurgie qui consiste à enlever le côlon et le rectum, ce qui est une intervention relativement lourde. En revanche, chez les sujets atteints par la maladie de Crohn, la chirurgie ne fait que traiter les complications mais n'empêche pas la maladie de réapparaître après que le patient ait été opéré.

La polyarthrite rhumatoïde (PR) est une maladie auto-immune, dont l'origine précise n'est pas connue ; elle est la cause la plus fréquente des polyarthrites chroniques. Elle représente le type de rhumatisme inflammatoire le plus rencontré chez l'adulte. On évalue sa prévalence entre 0,3 et 0,8 % selon les pays. Elle se caractérise par une atteinte articulaire souvent bilatérale et symétrique, évoluant par poussées vers la déformation et la destruction des articulations atteintes. La maladie débute généralement par une polyarthrite, c'est-à-dire l'inflammation de 4 ou plus articulations, caractérisée par des douleurs d'horaire inflammatoire (réveils nocturnes, dérouillage matinal >30 minutes), une raideur articulaire, et un gonflement appelé synovite. Le plus souvent, l'évolution, qui s'étale sur des dizaines d'années, se fait par poussées, entrecoupées de rémissions de rythme et de durée imprévisibles. Au cours des poussées, la plupart des articulations sont gonflées et douloureuses, associées à des signes généraux (fébricule, asthénie) et fréquemment d'un syndrome inflammatoire biologique. Le suivi de l'activité de la maladie peut se faire à l'aide de différents scores. Le plus utilisé en pratique clinique est le DAS 28, calculé à partir de 4 paramètres: l'indice articulaire (nombre d'articulations douloureuses - sauf pieds chevilles et hanches non comptabilisées), l'indice synovial (nombre d'articulations gonflées - sauf pieds chevilles et hanches), activité de la maladie évaluée sur une échelle de 0 à 100 par le patient, et vitesse de sédimentation. Le traitement symptomatique peut comporter le repos simple lors des poussées, les traitements antalgiques classiques, les anti-inflammatoires non stéroïdiens, les corticostéroïdes à faible dose, inférieure à 10 mg/jour pour en limiter les effets secondaires. Quant au traitement de fond et selon la sévérité de la maladie, il existe actuellement une panoplie de molécules qui peuvent être utilisées comme notamment le méthotrexate ; certains immunosuppresseurs comme l'azathioprime ou la ciclosporine ; les anti-TNF alpha ; un inhibiteur du CTLA4 ou les anti-CD20. Cependant, certaines de ces molécules doivent être utilisées avec précaution à cause d'effets secondaires importants qu'elles provoquent, ou ne sont pas suffisamment efficaces chez certains patients, ou alors doivent être prises en association.

La sclérose en plaques (SEP) quant à elle est une maladie inflammatoire du système nerveux central. Elle entraîne en particulier une démyélinisation inflammatoire, une destruction de la myéline dans la substance blanche de l'encéphale et de la moelle. Les lésions anciennes sont le siège d'une prolifération astrocytaire qui caractérise la sclérose du tissu nerveux. Ces lésions démyélinisantes ont une répartition et une topographie singulières, non pas diffuses mais en plaques. Les formes multiples que revêt la maladie la rendent difficilement compréhensible pour l'entourage aussi bien que complexe à diagnostiquer pour le corps médical. Actuellement, le traitement vise à limiter la fréquence et l'importance des poussées inflammatoires. Il comporte ainsi deux versants: un traitement de la poussée et un traitement de fond. Dans le traitement de la poussée, on prescrit parfois des corticostéroïdes en relai par voie orale pendant environ 3 semaines, associés à des mesures de prévention des effets secondaires des corticostéroïdes. En traitement de fond, l'interféron bêta et l'acétate de glatiramère (copolymère de plusieurs acides aminés) sont généralement prescrits. Dans les formes sévères, il peut être proposé d'utiliser des immunosupresseurs parmi lesquels la mitoxantrone. Des anticorps tels que le natalizumab (anticorps contre la chaîne α de l'intégrine des leucocytes) et le Remicade® (monoclonal anti-TNF-α) sont également prescrits.

Le psoriasis est une maladie de la peau d'origine mal connue, en partie génétique. Cette affection dermatologique touche 1 à 3 % de la population, aussi bien chez les femmes que chez les hommes.. Il existe plusieurs types de psoriasis dont le psoriasis en plaques ; le psoriasis en gouttes ; le psoriasis pustuleux ; l'arthrite psoriasique. Dans sa forme bénigne, le psoriasis se limite au cuir chevelu, aux ongles, aux genoux, aux coudes, aux pieds, aux mains et, parfois, aux organes génitaux. Dans les cas graves, il s'étend et peut gagner la totalité du corps. Cette dermatose chronique évolue de façon très individuelle, avec des poussées, mais aussi des rémissions au cours desquelles les lésions disparaissent. On dit alors que le psoriasis est «blanchi». Le répit est de durée très variable et la rémission souvent incomplète. A ce jour, aucun traitement curatif permettant de guérir complètement du psoriasis n'est connu ; il est toutefois possible de maîtriser le psoriasis, de diminuer l'étendue des lésions et d'améliorer la vie des patients. Il existe différents types de traitement :
- un traitement local pour la forme non sévère qui consiste à appliquer une crème à base de corticostéroides, calcipotriène ou de tazarotène sur la zone du psoriasis. Ces traitements ont un effet favorable sur le psoriasis, malheureusement les plaques reviennent souvent dès l'arrêt du traitement. Ce dernier engendre également une forme d'insensibilisation, qui oblige à augmenter les doses dans le temps. De plus, l'effet n'est plus seulement local si l'on applique ces pommades sur de vastes zones. Cette forme de traitement devrait donc être limitée à des formes aiguës ou fortement inesthétiques, pendant une courte période et sur une surface limitée ;
- un traitement de photothérapie. L'exposition solaire a le plus souvent une influence favorable sur le psoriasis. Cependant, dans 10% des cas, cette exposition sera en fait néfaste. Le sujet devra alors éviter le soleil, ou du moins éviter d'être directement exposé à ses rayons ;
- un traitement systémique. Pour les formes les plus sévères de psoriasis, les médecins peuvent prescrire des traitements par voie orale ou par injection. On utilise dans ce cas, le méthotrexate, la cyclosporine ou les anticorps anti-TNF-α. Ces traitements sont appelés systémiques car les médicaments sont sensés se disséminer dans tout l'organisme. Ils ont souvent des effets secondaires, parfois sérieux.

Il résulte de ces exemples que les traitements disponibles actuellement pour soigner les maladies autoimmunes sont en nombre limité et se révèlent parfois très lourds et inefficaces. Ceci explique l'importance de la recherche dans ce domaine. Plusieurs laboratoires pharmaceutiques et universitaires développent des médicaments ou traitements dans les maladies autoimmunes. On peut citer à titre d'exemple, des traitements à base de purines (FR2851248A et WO 96/18397A), d'anticorps contre les cytokines ou des récepteurs (US 2003232009A, WO 06/121852, WO 06/092530, EP 1593393A et WO 07/009065) de vaccins ou d'autoantigènes (WO 01/74375, EP 1621208A, WO 07/044394), etc. Ces molécules thérapeutiques sont encore en stade de recherche fondamentale ou clinique et il est difficile pour l'instant d'évaluer leur efficacité chez l'homme.

La Demanderesse s'est intéressée à un peptide analogue de la thymuline actif vis-à-vis du système immunitaire et du traitement des maladies autoimmunes. On connaissait depuis la fin des années 1950 le rôle central joué par le thymus dans la différenciation des lymphocytes T, responsables notamment du rejet des greffes et de la défense contre les virus et contre certaines bactéries. L'hormone, secrétée par le thymus fut alors identifiée comme un peptide de 9 acides aminés : la thymuline (Bach et al Pleau et al. Immunol letters, 1979;1:179-182; Amor et al., Annals of the Rheumatic Diseas 1987 :46 :549-554). Les propriétés de la thymuline sur le système immunitaire se sont révélées être zinc-dépendantes. En effet, le zinc associé au peptide confère à celui-ci une conformation tétraédrique qui correspond à la forme active de la molécule. En l'absence de zinc, la thymuline ne possède plus aucune activité vis-à-vis du système immunitaire.

L'utilisation possible de la thimuline dans le traitement des maladies autoimmunes tel que le Lupus érythémateux est suggéré dans US-A-4301065, US-A-4133804 et FR-A-2513125.

Des peptides analogues de la thymuline de la présente invention ont déjà été décrits pour le traitement de la douleur inflammatoire et neurogène (WO 03/030927) ; Saade et al, Neuroscience, 2003;119(1):155-65 et Safieh-Garabedian est al, Br J Pharmacol. 2002 Jul;136(6):947-55. Dans cette dernière publication, il est rapporté que le peptide PAT présente des actions analgésique et anti-inflammatoire, suite à une série d'expériences réalisées chez le rat en utilisant des injections d'endotoxines par voie intraplantaire (i.pl.) et par voie intrapéritonéale (i.p). Des dosages de cytokines ont été réalisés sur des prélèvements de tissus de peau et de foie. Il est important de préciser que ces auteurs ne se sont pas intéressés à la douleur inflammatoire intestinale ni à la douleur provenant de l'arthrite rhumatoïde. De plus, les modèles utilisés dans cette demande ne peuvent nullement être considérés comme des modèles pour étudier les maladies autoimmmunes et inflammatoires telles que les MICI (maladie de Crohn, rectocolite hémorragique) ou la polyarthrite rhumatoïde. En effet, le modèle de la capsaïcine a été utilisé dans cette étude comme modèle de douleur viscérale ; la capsaïcine est une molécule qui provoque des sensations de brûlure et de douleur. Il est important de préciser qu'elle agit au niveau des neurones sensoriels mais pas au niveau de l'inflammation proprement dite. Il est décrit dans un exemple que la capsaïcine est injectée à des rats qui ont reçu préalablement du peptide PAT, et ensuite les scores de nociception (perception douloureuse) provoquée par la capsaïcine sont relevés. Déjà par son modèle d'action, on peut constater que le modèle de la capsaïcine n'est pas adapté à l'étude des maladies autoimmunes telles que les MICI. De plus, la demande WO 03/030927 ne fait pas spécifiquement référence aux maladies autoimmmunes telles que les MICI comme la maladie de Crohn ou la rectocolite hémorragique.

La Demanderesse s'est intéressée à déterminer si le nonapeptide PAT - dont l'innocuité a été établie- pouvait être considéré comme un nouveau médicament pour traiter les maladies autoimmunes telles que les MICI, la polyarthrite rhumatoïde, la sclérose en plaque ou le psoriasis.

La présente invention se rapporte à l'utilisation du nonapeptide PAT répondant à la formule (I) :

EAKSQGGSD ;

ou un de ses sels pharmaceutiquement acceptables
dans la préparation d'un médicament dans le traitement des maladies autoimmunes telles que les maladies inflammatoires chroniques intestinales (MICI) ; la polyarthrite rhumatoïde, la sclérose en plaques et le psoriasis dont l'arthrite psoriasique.

La présente invention se rapporte tout particulièrement à l'utilisation thérapeutique du peptide PAT dans le traitement des MICI ; la maladie de Crohn. étant l'indication préférée.

Selon un autre mode de réalisation préféré, la présente invention concerne l'utilisation thérapeutique du peptide PAT dans le traitement de l'arihrite : la polyarthrite rhumatoïde étant l'indication préférée.

Le peptide PAT est administré chez l'homme ou chez l'animal à un dosage compris entre 0,1 et 50 mg ; et de préférence entre 1 et 10 mg.

Par " sel pharmaceutiquement acceptable", on entend par exemple et de manière non limitative un acétate, un sulfate ou un chlorhydrate.

L'invention concerne également l'utilisation d'un composé de formule (I) dans laquelle un ou plusieurs acides aminés sont en configuration D.

La composition pharmaceutique selon l'invention se présente sous une forme appropriée pour une administration :
- par voie parentérale, comme par exemple, sous forme de préparations injectables par voie intrapéritonéale, sous-cutanée, intraveineuse ou intramusculaire;
- par voie orale, comme par exemple, sous forme de comprimés enrobés ou non, de gélules, de poudres, de granulés, de suspensions ou de solutions orales. Une telle forme pour l'administration par voie orale peut être soit à libération immédiate, soit à libération prolongée ou retardée. De telles formes à libération prolongée ou retardée sont décrites, par exemple, dans les demandes EP 253104 ou EP 576 643 ;
- par voie rectale, comme par exemple, sous forme de suppositoires ;
- par voie topique, notamment transdermique, comme par exemple, sous la forme de " patch ", de pommade ou de gel.
- par voie intranasale, comme par exemple sous forme d'aérosols et " sprays »,
- par voie perlinguale,
- par voie intraoculaire.

Le véhicule pharmaceutiquement acceptable peut être choisi parmi les véhicules utilisés de manière classique selon chacun des modes d'administration.

D'autres avantages et caractéristiques de l'invention apparaîtront à la lecture des exemples qui suivent. Il sera fait référence aux dessins en annexe dans lesquels :
- la figure 1 représente l'effet du nonapeptide PAT sur le poids des animaux chez qui on a induit une colite par injection de TNBS ;
- la figure 2 illustre l'effet du nonapeptide PAT sur la diminution de l'inflammation du côlon causée par le TNBS ;
- la figure 3 représente l'effet du nonapeptide PAT sur la sécrétion des cytokines pro-inflammatoires TNF-α, IL-1β et IL-6 ;
- la figure 4 montre l'effet du peptide PAT (à 2 doses différentes) comparé à celui d'un AINS de référence (indométhacine) sur le volume de la patte des rats arthritiques ;
- la figure 5 montre l'effet du peptide PAT (à 2 doses différentes) comparé à celui d'un AINS de référence (indométhacine) sur le score clinique macroscopique chez des rats arthritiques ;
- la figure 6 montre l'effet du peptide PAT (à 2 doses différentes) comparé à celui d'un AINS de référence (indométhacine) sur l'hypersensibilité mesurée par le test plantaire chez des rats arthritiques ;
- la figure 7 montre l'effet du peptide PAT (à 2 doses différentes) comparé à celui d'un AINS de référence (indométhacine) sur la vitesse de sédimentation globulaire chez des rats arthritiques.

### EXEMPLE 1: EFFET DU PAT DANS UN MODELE DE LA MALADIE DE CROHN CHEZ LA SOURIS

### PROTOCOLE EXPERIMENTAL

### Synthèse du nonapeptide PAT

Le peptide a été assemblé sur phase solide selon une stratégie Foc/tu, clivé et déprotégé par l'acide trifluoroacétique, puis purifié par chromatographie haute pression préparative en phase inverse et lyophilisé. Sa pureté (>95%) et son identité furent confirmées par HPLC analytique et par spectrométrie de masse. Séquence du nonapeptide: (H- EAKSQGGSD-NH2 ; 877 Da)

Il est à noter que, lors de cette synthèse, une attention toute particulière a été portée pour réduire au maximum un produit de dégradation fréquent qui est la transformation de la glutamine (Glu) en pyro-glutamine (Pyro-Glu), ce qui entraîne la formation du peptide PyroGlu-Ala-Lys-Ser-Glu-Gly-Gly-Ser-Asp. Ce produit de dégradation a pu être réduit dans la présente invention grâce à l'optimisation des conditions de synthèse avec l'acétate, à la mise en solution du peptide dans du saccharose et au stockage du produit approprié (à -20°C). Le produit synthétisé contient 1,68% cette contamination.

### Induction de la colite

Le peptide PAT a été étudié chez l'animal dans un modèle de colite expérimentale. Chez la souris, un des modèles de MICI clairement établi est la colite induite par l'acide trinitro benzène sulfonique (TNBS). Le TNBS est un haptène qui, lorsqu'il est administré par voie intrarectale, induit une inflammation transmurale sévère du côlon ; les effets provoqués présentent beaucoup de similitudes avec ceux induits par la maladie de Crohn (Elson CO et al. Expérimentai models of inflammatory bowel disease. Gastroenterology 2003; 10:1344-67, Strober W et al, The immuiiology of mucosal models of inflammation. Annu Rev Immunol 2002;20:495-49).

La colite est induite chez les souris C57BL/6J âgées de 7-8 semaines après administration de TNBS par voie rectale selon le protocole décrit par Sugimoto K. et al. (Gastroenterology 2002: 123 ; 1912-1922). On administre a l'aide d'une seringue de 1 ml et d'une canule polyéthylène (Canules : Intradermic PE-20, Becton Dickinson) chez la souris 100µl d'une solution contenant 2,5 mg de TNBS/souris dissout dans 50µl d'éthanol directement dans la lumière du côlon (sur une longueur de 3 à 4cm). Après l'administration, les souris sont maintenues 30 secondes en position verticale.

### Traitement

Le design de l'étude est le suivant :
- un lot témoin de 5 souris contrôles traitées par l'éthanol /PBS (vol/vol) :
- un lot de 10 souris auxquelles on administre le TNBS (2,5 mg dans 50% éthanol) ;
- un lot de 10 souris auxquelles on administre le TNBS et le nonapeptide à la dose de 1µg/souris ;
- un lot de 10 souris auxquelles on administre le TNBS et le nonapeptide à la dose de 5µg /souris ;
- un lot de 10 souris auxquelles on administre le TNBS et le nonapeptide à la dose de 25µg /souris.

Le nonapeptide PAT est administré par voie intrapéritonéale 30 minutes avant l'induction de colite par le TNBS. Une partie des souris (5 pour chaque groupe des lots testés ; 2 pour le groupe des contrôles) sont sacrifiés le jour 1 après induction par le TNBS. L'autre partie (5 pour chaque groupe des lots testés, 3 pour le groupe des contrôles) sont sacrifiés le jour 3 après induction par le TNBS.

### Paramètres mesurés

### Poids des animaux:

Les animaux sont pesés tous les jours jusqu'au sacrifice.

### Inflammation du côlon

Dès que les animaux sont sacrifiés, le côlon est retiré et une analyse macroscopique est effectuée.

### Expression de IL-1β, IL-6 et TNF-α par RT-PCR quantitative

Aux jours 1 et 3 après induction par le TNBS, les animaux sont sacrifiés et les côlons sont prélevés pour qu'une extraction des ARN totaux soit effectuée.

Celle-ci est réalisée par dénaturation au thiocyanate de guanidine ; la synthèse des ADNc est effectuée à partir de 1 µg d'ARN à l'aide d'un kit (Euromedex). On réalise une PCR quantitative ; la réaction se déroule dans un volume final de 25 µl en utilisant le kit SYBR (Eurogentec). Le taux de stimulation est calculé pour chaque cytokine par rapport à l'ADNc de référence qui est celui correspondant à la phosphoprotéine ribosomale 34B4.

### RESULTATS

### Poids des animaux

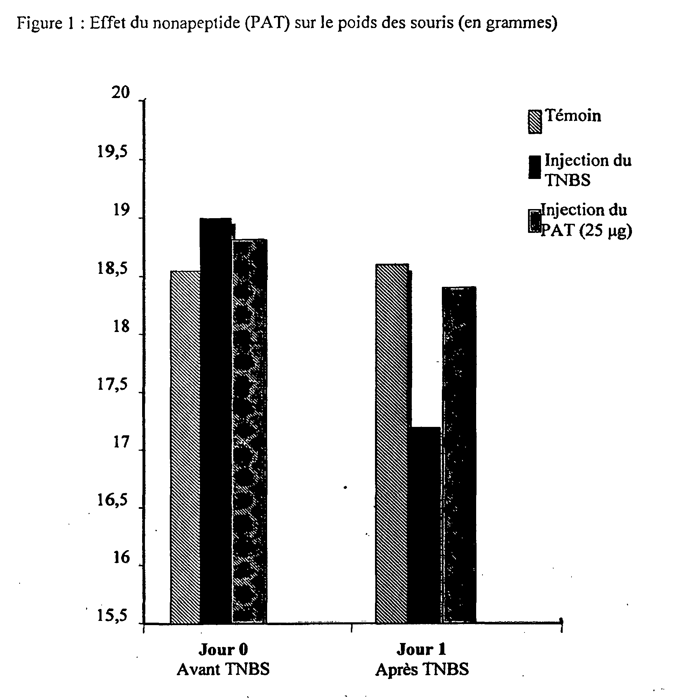

Les résultats obtenus dans la Figure 1 montre que l'injection du TNBS par voie rectale induit une diminution notable du poids des souris, et que l'administration préalable du nonapeptide PAT permet de limiter cette perte de poids surtout à la dose de 25 µg/souris. Les doses de 1 µg et de 5µg /souris n'ont pas eu d'effet significatif.

### Mesure de l'inflammation du côlon

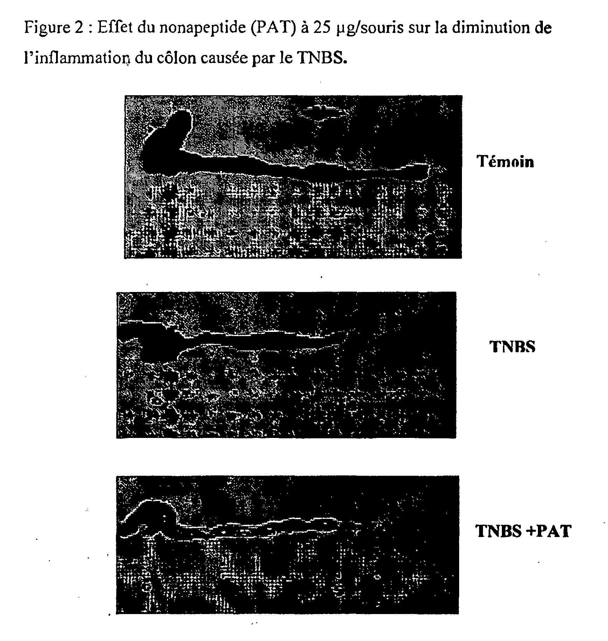

Il a été observé que le nonapeptide PAT permet de réduire de façon significative l'inflammation engendrée par l'injection rectale du TNBS. D'après les résultats montrés dans la Figure 2, cet effet est dose dépendant et l'effet maximum est observé pour la dose de 25 µg/souris. En moyenne, le côlon mesure 7 cm chez le rat témoin, 5 cm chez le rat traité par le TNBS, et 6,5 cm chez le rat traité par le TBNS et le peptide PAT.

### Dosage des cytokines proinflammatoires

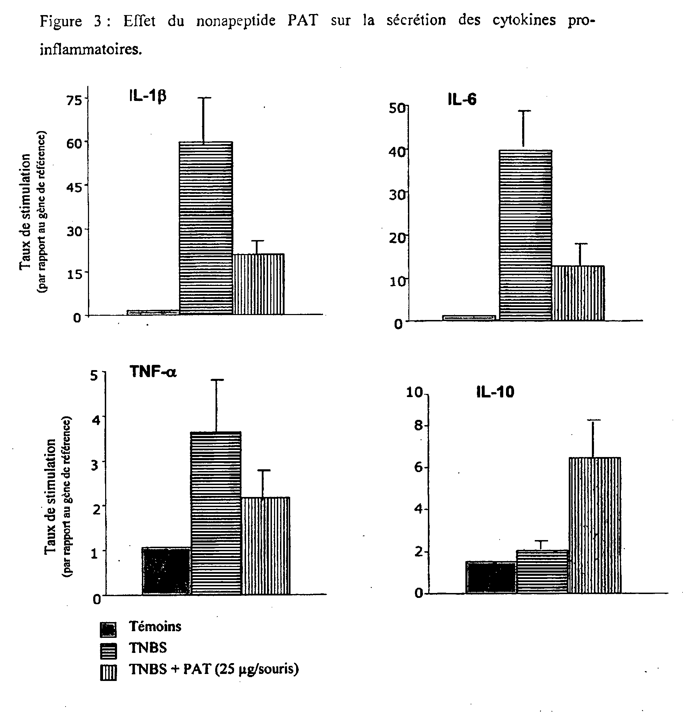

Dans la figure 3, on observe que l'expression de la cytokine IL-1β est augmentée en moyenne 50 fois chez les souris traitées au TNBS. Lorsqu'un traitement de 25 µg du peptide PAT est administré à la souris, on note que l'expression d'IL-1β diminue de façon significative.

L'expression de l'IL-6 est également fortement augmentée chez les souris traitées au TNBS (30 fois en moyenne). En présence du peptide PAT à 25 µg/souris, l'expression d'IL-1β diminue de façon significative.

En ce qui concerne le TNF-α, son expression augmente très légèrement (de 4 fois en moyenne) chez les souris auxquelles on a administré du TNBS. En présence du peptide PAT à une dose de 25 µg/souris, on observe que cette augmentation est nettement réduite.

De façon intéressante, nous avons noté chez les souris traitées au TNBS seule n'augmente pas la production de la cytokine anti-inflammatoire IL-10, alors que l'administration préalable du peptide PAT à une dose de 25 µg/souris provoque une augmentation significative de cette cytokine de plus de 3 fois.

Comme indiqué plus haut, le TNBS par voie intrarectale induit une inflammation sévère du côlon. Celle-ci présente beaucoup de similitude avec les symptômes induits par la maladie de Crohn et notamment au niveau de la sécrétion des cytokines pro-inflammatoires. Ces résultats associés aux précédents permettent d'affirmer que l'administration du nonapeptide PAT est un excellent candidat pour le traitement des maladies autoimmunes donc les MICI font partie.

### EXEMPLE 2: EFFET DU PAT DANS UN MODELE DE LA MALADIE DE L'ARTHRITE RHUMATOÏDE CHEZ LE RAT PROTOCOLE EXPERIMENTAL

### Modèle d'arthrite rhumatoïde

Nous avons utilisé le modèle d'arthrite induite par adjuvant de Freund tel que décrit dans l'article de Millan et al, J Neurosci 1986;6:899-906. Les rats ayant reçu le *Mÿcobacterium butyricum* développent généralement une polyarthrite sévère qui ressemble à celle qui se développe chez les humains. Les rats de la lignée OFA (SD) (Oncins France Strain A) pesant environ 200 g ont reçu une injection de 100 µl d'une solution de *Mycobacterium butyricum* à 10 mg/ml au niveau de l'articulation de cheville droite.

Pour tous les paramètres mesurés, les rats ont été répartis en 5 groupes (8 rats/groupe) de la façon suivante :
- groupe témoin : les rats ont reçu une injection de paraffine au lieu de la solution de *Mycobacterium butyricum* (pas d'arthrite induite) puis reçoivent une injection de solution saline par voie intra-péritonéale ;
- groupe arthritique + injection de solution saline ;
- groupe arthritique + injection d'indométhacine à 3 mg/kg par voie intra-péritonéale ; l'indométhacine étant un AINS couramment utilisé comme référence dans ce type de test ;
- groupe arthritique + injection de peptide PAT à 0,4 µg/kg par voie intra-péritonéale ;
- groupe arthritique + injection de peptide PAT à 4 µg/kg par voie intra-péritonéale.

Les produits sont administrés 5 fois par semaine pendant 3 semaines. Au début de l'expérimentation (jour 0), on détermine les valeurs de base concernant les divers paramètres (volume de la patte, score clinique macroscopique, vitesse de sédimentation et l'hypersensibilité).

### Paramètres mesurés

Au cours de l'expérience, plusieurs paramètres sont mesurés :
- le volume de la patte (« paw latency duration ») ;
- la vitesse de sédimentation globulaire (« *Erythrocyte Sédimentation Rate* ») et ;
- le score macroscopique :
   ▪ « 0 » signifie qu'aucun signe d'arthrite n'a été détecté ;
   ▪ « 1 » signifie qu'un gonflement ou une inflammation a été détecté au niveau d'une patte;
   ▪ « 2 » signifie qu'un gonflement ou une inflammation a été détecté au niveau de deux pattes;
   ▪ « 3 » signifie qu'un gonflement ou une inflammation a été détecté au niveau d'au moins 3 pattes;
   ▪ « 4 » signifie qu'un gonflement ou une inflammation a été détectée au niveau des 4 pattes.
- l'hypersensibilité grâce au test plantaire (« *Plantar test* ») : on réalise ce test selon Yukinori Nagakura et al., Allodynia and hyperalgesia in adjuvant-induced arthritic rats : time course of progression and efficace of analgesics, JPET 306 :490-497, 2003 ou dans Andersen ML et al. « Evaluation of acute and chronic treatments with Harpagophytum procumbens on Freund's adjuvant-induced arthritis in rats". J Ethnopharmacol. 2004 Apr;91(2-3):325-30;
- la vitesse de sédimentation globulaire (VSG) : l'augmentation de celle-ci accompagne généralement les symptômes cliniques de l'arthrite induite. On mesure la VSG chez les rats arthritiques (Jour=21) 1 heure et 2 heures après l'injection de PAT ou d'indométhacine, selon le protocole de Mahajan SG, et al, Protective Effect of Ethanolic Extract of Seeds of Moringa oleifera Lam. Against Inflammation Associated wlith Development of Arthritis in Rats. J Immunotoxicol. 2007 Jan;4(1):39-47.

### RESULATS

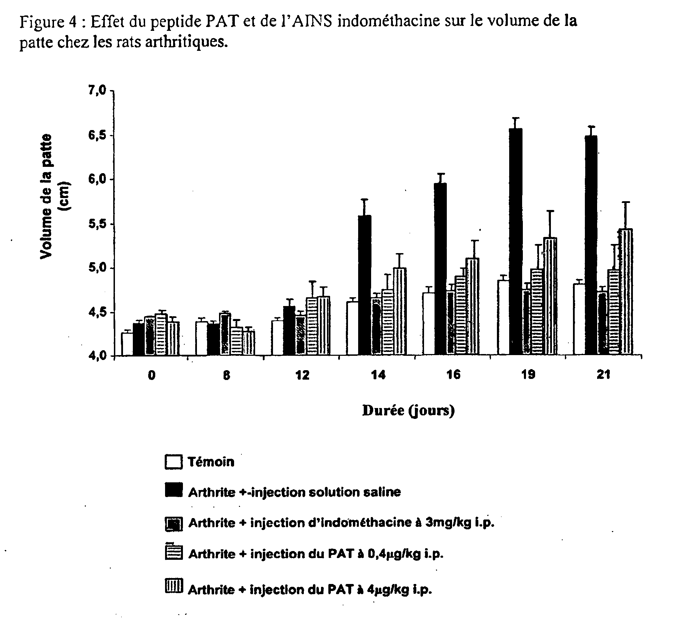

Dans la figure 4, on observe qu'à partir de 10 jours après l'administration de *Mycobacterium butyricum,* les rats commencent à montrer des signes d'inflammation. Chez les rats témoins, on n'observe pas de variation notable du volume de la patte (Figure 4). L'injection de l'adjuvant de Freund provoque un gonflement croissant de la patte et cela jusqu'au jour 19. Le traitement des animaux par administration du peptide PAT ou d'indométhacine n'a pas d'effet notable pendant les 12 premiers jours. En revanche, ces traitements provoquent à partir du jour 14 une diminution notable de l'inflammation ainsi que du volume de la patte ; cette diminution durant jusqu'au jour 21. De façon surprenante, il est à noter que le peptide PAT provoque le même effet aux deux doses 0,4 et 4 µg/kg suggérant qu'il n'y a pas d'effet dose-réponse.

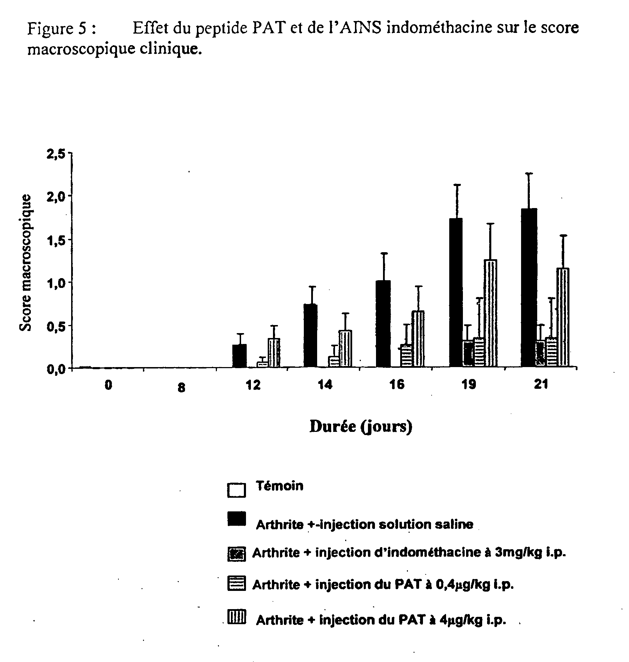

La figure 5 montre l'effet produit sur le score macroscopique clinique après administration du peptide PAT comparé à celui produit après administration d'indométhacine. On observe que la maladie est progressive chez les rats arthritiques qui ont reçu de la solution saline, et que et le score augmente jusqu'au jour 21. En revanche, on note une diminution significative du score macroscopique chez les rats arthritiques qui ont reçu le peptide PAT ou l'indométhacine lorsqu'on compare aux rats arthritiques qui ont reçu que de la solution saline. Comme observé précédemment, le peptide PAT administré à la dose de 4 µg/kg en intrapéritonéal montre moins d'effets que lorsqu'il est administré à la dose de 0.4 µg/kg.

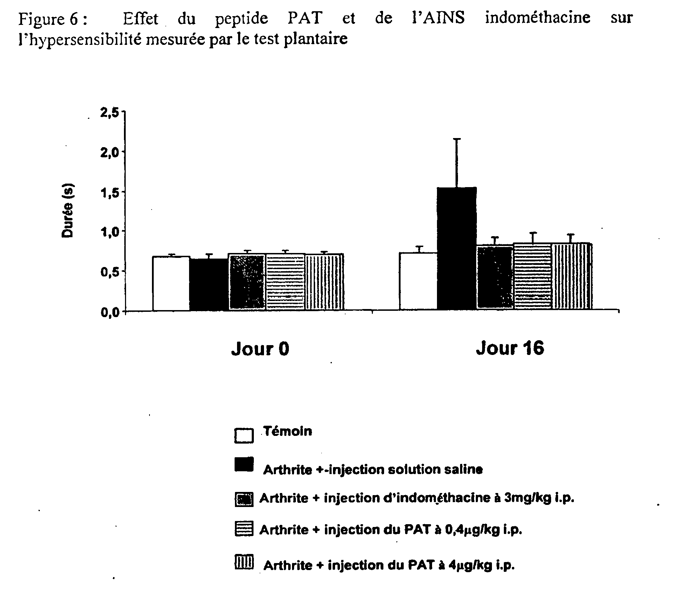

La figure 6 montre les résultats de l'effet du PAT sur l'hypersensibilité mesurée par le test plantaire chez les rats arthritiques. Au jour 0 ; la durée de latence est la même pour tous les groupes. Au jour 16, on observe une augmentation très significative de l'hypersensibilité dans le groupe des rats arthritiques non traités, alors que l'hypersensibilité est très réduite dans les groupes de rats arthritiques traités par le peptide PAT (aux 2 doses) et par l'indométhacine» ; cette hypersensibilité étant équivalente à celle du groupe témoin (non arthritique).

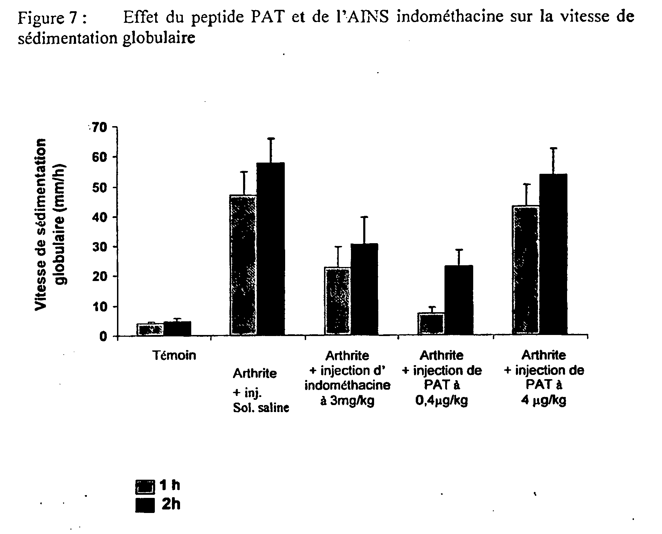

La figure 7 montre les résultats de la vitesse de sédimentation globulaire (VSG). Celle-ci-est assez élevée chez les rats arthritiques. En revanche, elle est réduite de façon très importante chez les rats arthritiques traités par le peptide PAT ou par l'AINS indométhacine. On remarque que le traitement à la dose de 0,4 µg/kg réduit davantage la VSG (lorsque celle est mesurée 1 heure après l'injection) que le traitement par l'indométhacine et s'approche de la VSG mesurée chez les rats témoins. Enfin le traitement par une dose 10 fois plus élevée de PAT (4 µg/kg) n'avait pas d'effet significatif sur la réduction de la VSG.

### CONCLUSIONS

Au vu des divers paramètres mesurés, les rats chez lesquels une arthrite est induite par le *Mycobacterium* développent généralement une polyarthrite sévère qui ressemble à celle qui se développe chez les humains. On peut constater que le peptide PAT, au moins à la dose de 0,4 µg/kg (i.p), permet de réduire très significativement la progression de l'arthrite et que son activité est détectable à divers niveaux corporels (patte ; hypersensibilité périphérique et vitesse de sédimentation sanguine). Enfin, il est intéressant de noter que le peptide PAT administré à la dose de 0,4 µg/kg (i.p.) a une efficacité similaire voir supérieure à l'AINS indométhacine administrée à la dose de 3 mg/kg, soit à une dose de 7500 fois inférieure par rapport à cette dernière.

## Revendications

1. Utilisation du nonapeptide PAT répondant à la formule (I) :
EAKSQGGSD ;
ou un de ses sels pharmaceutiquement acceptables
dans la préparation d'un médicament dans le traitement des maladies autoimmunes..

2. Utilisation du nonapeptide PAT selon la revendication 1, **caractérisée en ce que** la maladie autoimmune est une maladie inflammatoire chronique intestinale (MICI).

3. Utilisation du nonapeptide PAT selon la revendication 2, **caractérisée en ce que** la MICI est la maladie de Crohn.

4. Utilisation du nonapeptide PAT selon la revendication 1, **caractérisée en ce que** la maladie autoimmune est l'arthrite.

5. Utilisation du nonapeptide PAT selon la revendication 4, **caractérisée en ce que** l'arthrite est la polyarthrite rhumatoïde

6. Utilisation du nonapeptide PAT selon la revendication 1, **caractérisée en ce que** la maladie autoimmune est le psoriasis ou la sclérose en plaques.

7. Utilisation du nonapeptide PAT selon l'une quelconque des revendications précédentes, **caractérisée en ce qu'**un ou plusieurs acides aminés du peptide se présente en configuration D.

8. Utilisation du nonapeptide PAT selon l'une quelconque des revendications précédentes, **caractérisée en ce que** nonapeptide se présente sous une forme administrable par voie parentérale, par voie orale, par voie rectale ou par toute autre voie acceptable.

9. Utilisation du nonapeptide PAT selon la revendication 7 **caractérisée en ce que** la dose à administrer par voie parentérale est comprise entre 0,1 et 50 mg.

10. Utilisation du nonapeptide PAT selon l'une des revendications 8 ou 9, **caractérisée en ce que** la forme pharmaceutique appropriée contient un véhicule pharmaceutiquement acceptable.

## Patentansprüche

1. Verwendung des Nonapeptids PAT der Formel (I):
EAKSQGGSD;
oder eines seiner pharmazeutisch unbedenklichen Salze
bei der Herstellung eines Medikaments zur Behandlung von Autoimmunkrankheiten.

2. Verwendung des Nonapeptids PAT nach Anspruch 1, **dadurch gekennzeichnet, dass** es sich bei der Autoimmunkrankheit um eine chronisch-entzündliche Darmerkrankung (CED) handelt.

3. Verwendung des Nonapeptids PAT nach Anspruch 2, **dadurch gekennzeichnet, dass** es sich bei der CED um den Morbus Crohn handelt.

4. Verwendung des Nonapeptids PAT nach Anspruch 1, **dadurch gekennzeichnet, dass** es sich bei der Autoimmunkrankheit um Arthritis handelt.

5. Verwendung des Nonapeptids PAT nach Anspruch 4, **dadurch gekennzeichnet, dass** es sich bei der Arthritis um rheumatoide Arthritis handelt.

6. Verwendung des Nonapeptids PAT nach Anspruch 1, **dadurch gekennzeichnet, dass** es sich bei der Autoimmunkrankheit um Schuppenflechte oder multiple Sklerose handelt.

7. Verwendung des Nonapeptids PAT nach einem der vorhergehenden Ansprüche, **dadurch gekennzeichnet, dass** eine oder mehrere Aminosäuren des Peptids in D-Konfiguration vorliegen.

8. Verwendung des Nonapeptids PAT nach einem der vorhergehenden Ansprüche, **dadurch gekennzeichnet, dass** das Nonapeptid in einer parenteral, oral, rektal oder auf jede andere unbedenkliche Weise verabreichbaren Form vorliegt.

9. Verwendung des Nonapeptids PAT nach Anspruch 7, **dadurch gekennzeichnet, dass** die parenteral zu verabreichende Dosis zwischen 0,1 und 50 mg liegt.

10. Verwendung des Nonapeptids PAT nach einem der Ansprüche 8 oder 9, **dadurch gekennzeichnet, dass** die pharmazeutisch geeignete Form einen pharmazeutisch unbedenklichen Träger umfasst.

## Claims

1. Use of the PAT nonapeptide responding to the formula (I):
EAKSQGGSD ;
or one of its pharmaceutically acceptable salts
in the preparation of a drug in the treatment of autoimmune diseases.

2. Use of the PAT nonapeptide according to the claim 1, **characterized in that** the auto-immune disease is an a chronic inflammatory bowel disease (IBD).

3. Use of the PAT nonapeptide according to claim 2, **characterized in that** the IBD is the Crohn's disease.

4. Use of the PAT nonapeptide according to claim 1, **characterized in that** the auto-immune disease is the arthritis.

5. Use of the PAT nonapeptide according to claim 4, **characterized in that** the arthritis is the rheumatoid arthritis.

6. Use of the PAT nonapeptide according to claim 1, **characterized in that** the auto-immune disease is psoriasis or multiple sclerosis.

7. Use of the PAT nonapeptide according to any of the preceding claims, **characterized in that** one or several amino-acid of the peptide is (are) in configuration D.

8. Use of the PAT nonapeptide according to any of the preceding claims, **characterized in that** the nonapeptide is in a form administrable by parenteral route, oral route, rectal route or any other acceptable route.

9. Use of the PAT nonapeptide according to claim 7, **characterized in that** the dose to be administered is comprised between 0.1 and 50 mg.

10. Use of the PAT nonapeptide according to anyone of claim 8 or 9, **characterized in that** the suitable pharmaceutical form contains a pharmaceutically acceptable vehicle.


### SEQUENCE LISTING

<110> CLL PHARMA
<120> UTILISATION DU NONAPEPTIDE PAT DANS LE TRAITEMENT DE MALADIES AUTOIMMUNES
<130> CP-BB 63163-3574
<150> FR 08/02220
   <151> 2008-04-21
<150> pct/fr2009/000439
   <151> 2009-04-15
<160> 1
<170> PatentIn version 3.5
<210> 1
   <211> 9
   <212> PRT
   <213> Artificial Sequence
<220>
   <223> Peptide
<400> 1
   Glu Ala Lys Ser Gln Gly Gly Ser Asp
   1               5